# EUROPÄISCHE PATENTANMELDUNG

(11) **EP 4 822 009 A2**
(43) Veröffentlichungstag der Anmeldung: **14.10.2026**
(21) Anmeldenummer: 26153726.0
(22) Anmeldetag: 23.01.2026
(51) Int. Cl.: C12M 1/00, B01D 46/54, C12M 1/34

(54) **REDUZIERUNG DER CO2-KONZENTRATION IN EINEM CO2-INKUBATOR**

(30) Priorität: 23.01.2025 DE 102025102470
(71) Anmelder: Thermo Electron LED GmbH, 63505 Langenselbold (DE)
(72) Erfinder: Schneider, Jürgen, 63505 Langenselbold (DE); Barnkoth, Tino, 63505 Langenselbold (DE); Aulbach, Holger, 63505 Langenselbold (DE)
(74) Vertreter: Stellbrink & Partner Patentanwälte mbB

(57) **Zusammenfassung**

Die Erfindung betrifft einen CO₂-Inkubator umfassend einen Nutzraum, eine Druckausgleichsöffnung, die dazu ausgelegt ist, den Druck im Nutzraum an den Umgebungsdruck anzupassen, und eine CO₂-Versorgung, wobei der CO₂-Inkubator dazu ausgelegt ist, eine CO₂-Konzentration im Nutzraum zu reduzieren. Die Erfindung betrifft auch ein entsprechendes Verfahren zur Änderung einer CO₂-Konzentration in einem Nutzraum eines CO₂-Inkubators.

## Beschreibung

Die vorliegende Erfindung betrifft das Gebiet von CO₂-Inkubatoren und insbesondere die Inkubation von Zellen in Zellkultur- und Zelltherapieanwendungen unter Verwendung von großen Behältern zur Kultivierung einer großen Anzahl von Zellen in einem Inkubator.

In CO₂-Inkubatoren können Zellen während ihrer Wachstumsphase in großen Behältern (z.B. G-Rex^{®} Behälter) gelagert werden, insbesondere in Zellkultur- und Zelltherapieanwendungen. Während des Wachstums geben die lebenden Zellen CO₂ ab. Bei einer großen Anzahl von Zellen, die für die vorgenannten Anwendungen typisch ist, kann der CO₂-Ausstoß zu einem erheblichen Anstieg der CO₂-KOnzentration innerhalb des CO₂-Inkubators führen, sodass diese den eigentlichen Sollwert übersteigen kann. Dieser Anstieg kann durch bekannte CO₂-Inkubatoren typischerweise nicht kompensiert werden, sodass sich die CO₂-Konzentration und damit der pH-Wert im Medium kritisch verändern kann. Dies kann zur Beschädigung oder schlimmstenfalls zum vollständigen Verlust der Zellen führen.

Bekannte CO₂-Inkubatoren haben üblicherweise keine Möglichkeit, die CO₂-Konzentration kontrolliert zu verringern. Insbesondere kann die CO₂-Regelung zwar CO₂ hinzufügen, indem CO2-Gas in das Gerät eingeleitet wird, eine Vorrichtung zur kontrollierten Reduzierung der CO₂-Konzentration innerhalb des Gerätes ist aber typischerweise nicht verfügbar. Zwar können Nutzer zur Reduzierung der CO₂-Konzentration die Tür des CO₂-Inkubators öffnen. Dies führt jedoch nachteilig dazu, dass sich auch die Temperatur und die Feuchtigkeit den Werten der das Gerät umgebenden Atmosphäre annähern und entsprechend zumindest vorrübergehend von den entsprechenden Sollwerten abweichen.

Vor diesem Hintergrund ist es eine Aufgabe der vorliegenden Erfindung, die Unzulänglichkeiten und Nachteile des Standes der Technik zu überwinden oder zumindest zu reduzieren. Allgemein kann es Aufgabe der vorliegenden Erfindung sein, eine aktive Reduzierung der CO₂- Konzentration innerhalb des CO₂-Inkubators zu ermöglichen.

Die Aufgabe wird durch die Gegenstände der unabhängigen Patentansprüche gelöst. Vorteilhafte Weiterbildungen der Erfindung sind durch die abhängigen Patentansprüche, die folgende Beschreibung sowie die Figuren beschrieben.

Gemäß einem ersten Aspekt betrifft die Erfindung einen CO₂-Inkubator umfassend einen Nutzraum, eine Druckausgleichsöffnung, die dazu ausgelegt ist, den Druck im Nutzraum an den Umgebungsdruck anzupassen, und eine CO₂-Versorgung, wobei der CO₂-Inkubator dazu ausgelegt ist, eine CO₂-Konzentration im Nutzraum zu reduzieren.

Der erfindungsgemäße CO₂-Inktubator kann also insbesondere die CO₂-Konzentration im Nutzraum reduzieren. Dadurch kann vorteilhaft ein ungewünschter Anstieg der CO₂-Konzentration im Nutzraum verhindert werden. Ein entsprechender kann beispielsweise durch eine große Anzahl von Zellen hervorgerufen werden, die während ihrem Wachstum CO₂ abgeben.

In Ausführungsformen der Erfindung kann vorgesehen sein, dass der Inkubator dazu ausgelegt ist, dem Nutzraum kontrolliert Zuluft zuzuführen.

In Ausführungsformen der Erfindung kann vorgesehen sein, dass der Inkubator dazu ausgelegt ist Zuluft aktiv in den Nutzraum einzubringen.

Alternativ kann in Ausführungsformen der Erfindung vorgesehen sein, dass der Inkubator dazu ausgelegt ist aktiv Luft aus dem Nutzraum abzusaugen und dadurch über die Druckausgleichsöffnung Zuluft zuzuführen.

In Ausführungsformen der Erfindung kann vorgesehen sein, dass die Druckausgleichsöffnung einen Druckausgleichsfilter umfasst, der vorzugsweise einen Sinterfilter umfasst.

In Ausführungsformen der Erfindung kann vorgesehen sein, dass der Inkubator eine Pumpe umfasst.

In Ausführungsformen der Erfindung kann vorgesehen sein, dass die Pumpe dazu ausgelegt ist Luft aus dem Nutzraum abzusaugen.

Es versteht sich, dass der Begriff "Luft" sich in diesem Zusammenhang auf das Gas- und/oder Aerosolgemisch bezieht, welches sich im Nutzraum befindet.

In Ausführungsformen der Erfindung kann vorgesehen sein, dass ein Einlass der Pumpe fluidisch mit dem Nutzraum verbunden ist.

In Ausführungsformen der Erfindung, in denen der Inkubator dazu ausgelegt ist aktiv Luft aus dem Nutzraum abzusaugen, kann vorgesehen sein, dass der Inkubator ein Abluftventil umfasst, welches fluidisch mit dem Nutzraum und einem Abluftreservoir verbunden und zwischen diesen angeordnet ist, wobei das Abluftreservoir einen niedrigeren Druck als der Nutzraum aufweist.

In Ausführungsformen der Erfindung kann vorgesehen sein, dass die Pumpe dazu ausgelegt ist Zuluft in den Nutzraum zu fördern.

In Ausführungsformen der Erfindung kann vorgesehen sein, dass ein Auslass der Pumpe fluidisch mit dem Nutzraum verbunden ist.

In Ausführungsformen der Erfindung kann vorgesehen sein, dass der Inkubator ferner einen Zuluftfilter umfasst, der stromabwärts der Pumpe angeordnet ist.

In Ausführungsformen der Erfindung kann vorgesehen sein, dass ein Einlass der Pumpe mit einem Zuluftreservoir verbunden ist.

In Ausführungsformen der Erfindung kann vorgesehen sein, dass der Inkubator einen Luftkanal umfasst, wobei der Luftkanal umfasst:
einen Einlass und einen Auslass, die jeweils mit dem Nutzraum verbunden sind,
einen Ventilator, der dazu ausgelegt ist Luft vom Einlass zum Auslass des Luftkanals zu fördern, und
einem Strömungswiderstand, der stromaufwärts des Ventilators und stromabwärts des Einlasses angeordnet ist;
wobei zwischen dem Strömungswiderstand dem Ventilator (52) eine Zuluftleitung angeordnet ist, die mit einem Zuluftreservoir verbunden ist.

Weiterhin kann vorgesehen sein, dass der Luftkanal so ausgelegt ist, dass zwischen dem Strömungswiderstand und dem Ventilator ein Unterdruck erzeugt werden kann. Der Unterdruck kann mindestens bis zu 0.5 mbar, vorzugsweise mindestens bis zu 1 mbar unter atmosphärischem Druck betragen.

In Ausführungsformen der Erfindung kann vorgesehen sein, dass der Strömungswiderstand ein Luftkanalfilter, vorzugsweise ein Schwebstofffilter, weiter bevorzugt ein HEPA-Filter ist.

In Ausführungsformen der Erfindung kann vorgesehen sein, dass in der Zuluftleitung ein Zuluftfilter angeordnet ist.

In Ausführungsformen der Erfindung kann vorgesehen sein, dass in der Zuluftleitung ein Zuluftventil angeordnet ist.

In Ausführungsformen der Erfindung kann vorgesehen sein, dass das Zuluftventil stromabwärts vom Zuluftfilter angeordnet ist.

In Ausführungsformen der Erfindung kann vorgesehen sein, dass das Zuluftventil ein Proportionalventil ist.

In Ausführungsformen der Erfindung kann vorgesehen sein, dass der Luftkanal einen Befeuchter umfasst, der stromaufwärts vom Strömungswiderstand angeordnet ist.

In Ausführungsformen der Erfindung kann vorgesehen sein, dass der Inkubator einen CO₂-Sensor umfasst, der dazu ausgelegt ist, die CO₂-Konzentration im Nutzraum zu bestimmen.

In Ausführungsformen der Erfindung kann vorgesehen sein, dass der Inkubator einen Controller umfasst.

In Ausführungsformen der Erfindung kann vorgesehen sein, dass der Controller dazu ausgelegt ist, die CO₂-Konzentration im Nutzraum zu reduzieren, wenn ein CO₂-Grenzwert überschritten wird.

In Ausführungsformen der Erfindung kann vorgesehen sein, dass der Controller ausgelegt ist, die CO₂-Konzentration im Nutzraum bezüglich eines vorgegebenen Sollwertes zu steuern und/oder zu regeln.

In Ausführungsformen der Erfindung kann vorgesehen sein, dass der Controller ausgelegt ist, das Zuluftventil und optional den Ventilator zu steuern und/oder zu regeln.

In Ausführungsformen der Erfindung kann vorgesehen sein, dass die Pumpe eine Luftpumpe oder eine Vakuumpumpe ist

In Ausführungsformen der Erfindung kann vorgesehen sein, dass der Controller ausgelegt ist, die Pumpe zu steuern und/oder zu regeln.

In Ausführungsformen der Erfindung kann vorgesehen sein, dass der Zuluftfilter ein Membranfilter ist.

In Ausführungsformen der Erfindung kann vorgesehen sein, dass der Zuluftfilter ein Sterilfilter, vorzugsweise aus PTFE, ist.

In Ausführungsformen der Erfindung kann vorgesehen sein, dass die CO₂-Versorgung ein CO₂-Ventil und einen CO₂-Filter umfasst und dazu ausgelegt ist mit einem CO₂-Reservoir verbunden zu werden.

In Ausführungsformen der Erfindung kann vorgesehen sein, dass der CO₂-Filter stromaufwärts vom CO₂-Ventil angeordnet ist.

In Ausführungsformen der Erfindung kann vorgesehen sein, dass das CO₂-Ventil ein Proportionalventil ist.

In Ausführungsformen der Erfindung kann vorgesehen sein, dass der Inkubator ausgelegt ist, dass die Pumpe nicht läuft, wenn das CO₂-Ventil geöffnet ist.

In Ausführungsformen der Erfindung kann vorgesehen sein, dass der CO₂-Filter ein Membranfilter ist.

In Ausführungsformen der Erfindung kann vorgesehen sein, dass der CO₂-Filter ein Sterilfilter, vorzugsweise aus PTFE, ist.

In Ausführungsformen der Erfindung kann vorgesehen sein, dass die Zuluft mindestens eins aus Umluft des Inkubators, Sauerstoff und/oder Stickstoff ist.

In Ausführungsformen der Erfindung kann vorgesehen sein, dass das Zuluftreservoir durch die Umgebung des CO₂-Inkubators bereitgestellt wird.

Nach einem weiteren Aspekt ist die Erfindung auf ein Verfahren zur Änderung einer CO₂-Konzentration in einem Nutzraum eines CO₂-Inkubators gerichtet, wobei das Verfahren umfasst: Bestimmen der CO₂-Konzentration im Nutzraum, Vergleichen der CO₂-Konzentration mit einem vorgegebenen CO₂-Wert, Reduzieren der CO₂-Konzentration im Nutzraum des CO₂-Inkubators, wenn der CO₂-Wert überschritten wird.

In Ausführungsformen der Erfindung kann vorgesehen sein, dass der vorgegebene CO₂-Wert ein CO₂-Grenzwert ist.

In Ausführungsformen der Erfindung kann vorgesehen sein, dass der vorgegebene CO₂-Wert ein CO₂-Sollwert ist.

In Ausführungsformen der Erfindung kann vorgesehen sein, dass das Bestimmen der CO₂-Konzentration ein Messen der CO₂-Konzentration in der Luft innerhalb des CO₂-Inkubators umfasst.

In Ausführungsformen der Erfindung kann vorgesehen sein, dass wobei das Reduzieren der CO₂-Konzentration im Nutzraum das aktive Zuführen von Zuluft in den Nutzraum umfasst.

In Ausführungsformen der Erfindung kann vorgesehen sein, dass das aktive Zuführen von Zuluft das Absaugen von Luft aus den Nutzraum umfasst, um so Zuluft beispielsweise über eine Druckausgleichsöffnung nachströmen zu lassen.

In Ausführungsformen der Erfindung kann vorgesehen sein, dass das aktive Zuführen von Zuluft das Pumpen von Zuluft in den Nutzraum umfasst.

In Ausführungsformen der Erfindung kann vorgesehen sein, dass das aktive Zuführen von Zuluft das Ansaugen von Zuluft über eine mit einem Luftkanal des Inkubators verbundenen Zuluftleitung mittels Unterdruck umfasst.

In Ausführungsformen der Erfindung kann vorgesehen sein, dass das Verfahren weiterhin Filtern der Zuluft umfasst.

In Ausführungsformen der Erfindung kann vorgesehen sein, dass die Zuluft aus einem Zuluftreservoir zugeführt wird.

Das Zuluftreservoir kann beispielsweise die Umgebung des CO₂-Unkubators sein, sodass die Zuluft der Umgebungsluft entspricht. Alternativ kann das Zuluftreservoir auch durch einen Behälter, bspw. eine Gasflasche bereitgestellt werden, sodass die Zusammensetzung der Zuluft genau kontrolliert werden kann.

In Ausführungsformen der Erfindung kann vorgesehen sein, dass das Verfahren weiterhin umfasst: Erhöhen der CO₂-Konzentration im Nutzraum des CO₂-Inkubators, wenn der CO₂-Wert unterschritten wird.

In Ausführungsformen der Erfindung kann vorgesehen sein, dass das Verfahren das Empfangen des CO₂-Wertes umfasst.

In Ausführungsformen der Erfindung kann vorgesehen sein, dass das Verfahren das Steuern oder Regeln der CO₂-Konzentration in Abhängigkeit des CO₂-Wertes umfasst.

In Ausführungsformen der Erfindung kann vorgesehen sein, dass das Regeln der CO₂-Konzentration das Bestimmen, Vergleichen, Erhöhen und Reduzieren der CO₂-Konzentration im Nutzraum umfasst.

In Ausführungsformen der Erfindung kann vorgesehen sein, dass der CO₂-Inkubator ein CO₂-Inkubator nach einer der vorstehend beschriebenen Inkubator-Ausführungsformen ist.

In Ausführungsformen der Erfindung kann vorgesehen sein, dass der Inkubator ausgelegt ist das Verfahren nach einer der vorstehend beschriebenen Verfahrens-Ausführungsformen durchzuführen.

In Ausführungsformen der Erfindung kann vorgesehen sein, dass der Controller ausgelegt ist das Verfahren nach einer der vorstehend beschriebenen Verfahrens-Ausführungsformen durchzuführen.

Die Erfindung wird auch durch die folgenden nummerierten Ausführungsformen definiert.

Nachfolgend wird auf Inkubator-Ausführungsformen Bezug genommen. Diese Ausführungsformen sind durch ein I gefolgt von einer Zahl gekennzeichnet. Wenn im Folgenden von Inkubator-Ausführungsformen oder auch I-Ausführungsformen die Rede ist, sind diese Ausführungsformen gemeint.

I1. CO₂-Inkubator umfassend
einen Nutzraum (11),
eine Druckausgleichsöffnung (3), die dazu ausgelegt ist, den Druck im Nutzraum (11) an den Umgebungsdruck anzupassen, und
eine CO₂-Versorgung (2),
wobei der CO₂-Inkubator dazu ausgelegt ist, eine CO₂-Konzentration im Nutzraum (11) zu reduzieren.

I2. Inkubator nach der vorstehenden Inkubator-Ausführungsform, wobei der Inkubator dazu ausgelegt ist, dem Nutzraum (11) kontrolliert Zuluft zuzuführen.

I3. Inkubator nach einer der vorstehenden Inkubator-Ausführungsformen, wobei der Inkubator dazu ausgelegt ist Zuluft aktiv in den Nutzraum (11) einzubringen.

I4. Inkubator nach der vorletzten Inkubator-Ausführungsform, wobei der Inkubator dazu ausgelegt ist aktiv Luft aus dem Nutzraum (11) abzusaugen und dadurch über die Druckausgleichsöffnung (3) Zuluft zuzuführen.

I5. Inkubator nach einer der vorstehenden Inkubator-Ausführungsformen, wobei die Druckausgleichsöffnung (3) einen Druckausgleichsfilter (32) umfasst, der vorzugsweise einen Sinterfilter umfasst.

I6. Inkubator nach einer der vorstehenden Inkubator-Ausführungsformen, wobei der Inkubator eine Pumpe (41) umfasst.

I7. Inkubator nach der vorstehenden Inkubator-Ausführungsform soweit nicht auf Ausführungsform I3 rückbezogen, wobei die Pumpe (41) dazu ausgelegt ist Luft aus dem Nutzraum (11) abzusaugen.

Es versteht sich, dass der Begriff "Luft" sich in diesem Zusammenhang auf das Gas- und/oder Aerosolgemisch bezieht, welches sich im Nutzraum befindet.

I8. Inkubator nach der vorstehenden Inkubator-Ausführungsform, wobei ein Einlass der Pumpe (41) fluidisch mit dem Nutzraum (11) verbunden ist.

I9. Inkubator nach einer der vorstehenden Inkubator-Ausführungsformen mit den Merkmalen der Ausführungsform I4, wobei der Inkubator ein Abluftventil umfasst, welches fluidisch mit dem Nutzraum (11) und einem Abluftreservoir verbunden und zwischen diesen angeordnet ist, wobei das Abluftreservoir einen niedrigeren Druck als der Nutzraum aufweist.

I10. Inkubator nach einer der vorstehenden Inkubator-Ausführungsformen mit den Merkmalen der Ausführungsform I6 soweit nicht auf Ausführungsform I4 rückbezogen, wobei die Pumpe (41) dazu ausgelegt ist Zuluft in den Nutzraum (11) zu fördern.

I11. Inkubator nach der vorstehenden Inkubator-Ausführungsform, wobei ein Auslass der Pumpe (41) fluidisch mit dem Nutzraum verbunden ist.

I12. Inkubator nach einer der 2 vorstehenden Inkubator-Ausführungsformen, wobei der Inkubator ferner einen Zuluftfilter (43) umfasst, der stromabwärts der Pumpe (41) angeordnet ist.

I13. Inkubator nach einer der 3 vorstehenden Inkubator-Ausführungsform, wobei ein Einlass der Pumpe (41) mit einem Zuluftreservoir verbunden ist.

I14. Inkubator nach einer der vorstehenden Inkubator-Ausführungsformen, wobei der Inkubator einen Luftkanal (5) umfasst, wobei der Luftkanal (5) umfasst:
einen Einlass und einen Auslass, die jeweils mit dem Nutzraum (11) verbunden sind,
einen Ventilator (52), der dazu ausgelegt ist Luft vom Einlass zum Auslass des Luftkanals (5) zu fördern, und
einem Strömungswiderstand, der stromaufwärts des Ventilators (52) und stromabwärts des Einlasses angeordnet ist;
wobei zwischen dem Strömungswiderstand dem Ventilator (52) eine Zuluftleitung (44) angeordnet ist, die mit einem Zuluftreservoir verbunden ist.

I15. Inkubator nach der vorstehenden Inkubator-Ausführungsform, wobei der Luftkanal so ausgelegt ist, dass zwischen dem Strömungswiderstand und dem Ventilator (52) ein Unterdruck erzeugt werden kann.

I16. Inkubator nach der vorstehenden Inkubator-Ausführungsform, wobei der Unterdruck mindestens bis zu 0.5 mbar, vorzugsweise mindestens bis zu 1 mbar unter atmosphärischem Druck betragen kann.

I17. Inkubator nach einer der 3 vorstehenden Inkubator-Ausführungsformen, wobei der Strömungswiderstand ein Luftkanalfilter (53), vorzugsweise ein Schwebstofffilter, weiter bevorzugt ein HEPA-Filter ist.

I18. Inkubator nach einer der 4 vorstehenden Inkubator-Ausführungsformen, wobei in der Zuluftleitung (44) ein Zuluftfilter (43) angeordnet ist.

I19. Inkubator nach einer der 4 vorstehenden Inkubator-Ausführungsformen, wobei in der Zuluftleitung (44) ein Zuluftventil (45) angeordnet ist.

I20. Inkubator nach der vorstehenden Inkubator-Ausführungsform und mit den Merkmalen der Ausführungsform I18, wobei das Zuluftventil (45) stromabwärts vom Zuluftfilter (43) angeordnet ist.

I21. Inkubator nach einer der 2 vorstehenden Inkubator-Ausführungsformen, wobei das Zuluftventil (45) ein Proportionalventil ist.

I22. Inkubator nach einer der 8 vorstehenden Inkubator-Ausführungsformen, wobei der Luftkanal (5) einen Befeuchter (51) umfasst, der stromaufwärts vom Strömungswiderstand (53) angeordnet ist.

I23. Inkubator nach einer der vorstehenden Inkubator-Ausführungsformen, wobei der Inkubator einen CO₂-Sensor umfasst, der dazu ausgelegt ist, die CO₂-Konzentration im Nutzraum (11) zu bestimmen.

I24. Inkubator nach einer der vorstehenden Inkubator-Ausführungsformen, wobei der Inkubator einen Controller umfasst.

I25. Inkubator nach der vorstehenden Inkubator-Ausführungsform, wobei der Controller dazu ausgelegt ist, die CO₂-Konzentration im Nutzraum (11) zu reduzieren, wenn ein CO₂-Grenzwert überschritten wird.

I26. Inkubator nach der 2 vorstehenden Inkubator-Ausführungsformen, wobei der Controller ausgelegt ist, die CO₂-Konzentration im Nutzraum (11) bezüglich eines vorgegebenen Sollwertes zu steuern und/oder zu regeln.

I27. Inkubator nach einer der vorstehenden Inkubator-Ausführungsform en und mit den Merkmalen der Ausführungsform I19 und I24, wobei der Controller ausgelegt ist, das Zuluftventil (45) und optional den Ventilator (52) zu steuern und/oder zu regeln.

I28. Inkubator nach einer der vorstehenden Inkubator-Ausführungsformen und mit den Merkmalen der Ausführungsform I6, wobei die Pumpe eine Luftpumpe oder eine Vakuumpumpe ist

I29. Inkubator nach einer der vorstehenden Inkubator-Ausführungsformen und mit den Merkmalen der Ausführungsform I6 und I24, wobei der Controller ausgelegt ist, die Pumpe zu steuern und/oder zu regeln.

I30. Inkubator nach einer der vorstehenden Inkubator-Ausführungsformen und mit den Merkmalen mindestens einer der Ausführungsformen I12 oder I18, wobei der Zuluftfilter ein Membranfilter ist.

I31. Inkubator nach einer der vorstehenden Inkubator-Ausführungsformen und mit den Merkmalen mindestens einer der Ausführungsformen I12 oder I18, wobei der Zuluftfilter ein Sterilfilter, vorzugsweise aus PTFE, ist.

I32. Inkubator nach einer der vorstehenden Inkubator-Ausführungsformen, wobei die CO₂-Versorgung (2) ein CO₂-Ventil (22) und einen CO₂-Filter (23) umfasst und dazu ausgelegt ist mit einem CO₂-Reservoir (21) verbunden zu werden.

I33. Inkubator nach der vorstehenden Inkubator-Ausführungsform, wobei der CO₂-Filter (23) stromaufwärts vom CO₂-Ventil (22) angeordnet ist.

I34. Inkubator nach einer der 2 vorstehenden Inkubator-Ausführungsformen, wobei das CO₂-Ventil ein Proportionalventil ist.

I35. Inkubator nach einer der 3 vorstehenden Inkubator-Ausführungsformen und mit den Merkmalen der Ausführungsform I6, wobei der Inkubator ausgelegt ist, dass die Pumpe nicht läuft, wenn das CO₂-Ventil geöffnet ist.

I36. Inkubator nach einer der 4 vorstehenden Inkubator-Ausführungsformen, wobei der CO₂-Filter (23) ein Membranfilter ist.

I37. Inkubator nach einer der 5 vorstehenden Inkubator-Ausführungsformen, wobei der CO₂-Filter (23) ein Sterilfilter, vorzugsweise aus PTFE, ist.

I38. Inkubator nach einer der vorstehenden Inkubator-Ausführungsformen, wobei die Zuluft mindestens eins aus Umluft des Inkubators, Sauerstoff und/oder Stickstoff ist.

I39. Inkubator nach einer der vorstehenden Inkubator-Ausführungsformen und mit den Merkmalen von I13 oder I14, wobei das Zuluftreservoir durch die Umgebung des CO₂-Inkubators bereitgestellt wird.

Nachfolgend wird auf Verfahrens-Ausführungsformen Bezug genommen. Diese Ausführungsformen sind durch ein V gefolgt von einer Zahl gekennzeichnet. Wenn im Folgenden von Verfahrens-Ausführungsformen oder auch V-Ausführungsformen die Rede ist, sind diese Ausführungsformen gemeint.

V1. Verfahren zur Änderung einer CO₂-Konzentration in einem Nutzraum eines CO₂-Inkubators, wobei das Verfahren umfasst:
Bestimmen der CO₂-Konzentration im Nutzraum,
Vergleichen der CO₂-Konzentration mit einem vorgegebenen CO₂-Wert,
Reduzieren der CO₂-Konzentration im Nutzraum des CO₂-Inkubators, wenn der CO₂-Wert überschritten wird.

V2. Verfahren nach der vorstehenden Verfahrens-Ausführungsform, wobei der vorgegebene CO₂-Wert ein CO₂-Grenzwert ist.

V3. Verfahren nach der vorletzten Verfahrens-Ausführungsform, wobei der vorgegebene CO₂-Wert ein CO₂-Sollwert ist.

V4. Verfahren nach einer der vorstehenden Verfahrens-Ausführungsformen, wobei das Bestimmen der CO₂-Konzentration ein Messen der CO₂-Konzentration in der Luft innerhalb des CO₂-Inkubators umfasst.

V5. Verfahren nach einer der vorstehenden Verfahrens-Ausführungsformen, wobei das Reduzieren der CO₂-Konzentration im Nutzraum das aktive Zuführen von Zuluft in den Nutzraum umfasst.

V6. Verfahren nach der vorstehenden Verfahrens-Ausführungsform, wobei das aktive Zuführen von Zuluft das Absaugen von Luft aus den Nutzraum umfasst, um so Zuluft beispielsweise über eine Druckausgleichsöffnung nachströmen zu lassen.

V7. Verfahren nach der vorletzten Verfahrens-Ausführungsform, wobei das aktive Zuführen von Zuluft das Pumpen von Zuluft in den Nutzraum umfasst.

V8. Verfahren nach einer der vorstehenden Verfahrens-Ausführungsformen und mit den Merkmalen der Verfahrens-Ausführungsform V5, wobei das aktive Zuführen von Zuluft das Ansaugen von Zuluft über eine mit einem Luftkanal des Inkubators verbundenen Zuluftleitung mittels Unterdruck umfasst.

V9. Verfahren nach einer der vorstehenden Verfahrensausführungsformen und mit den Merkmalen der Verfahrens-Ausführungsform V5, wobei das Verfahren weiterhin Filtern der Zuluft umfasst.

V10. Verfahren nach einer der vorstehenden Verfahrensausführungsformen und mit den Merkmalen der Verfahrens-Ausführungsform V5, wobei die Zuluft aus einem Zuluftreservoir zugeführt wird.

Das Zuluftreservoir kann beispielsweise die Umgebung des CO₂-Unkubators sein, sodass die Zuluft der Umgebungsluft entspricht. Alternativ kann das Zuluftreservoir auch durch einen Behälter, bspw. eine Gasflasche bereitgestellt werden, sodass die Zusammensetzung der Zuluft genau kontrolliert werden kann.

V11. Verfahren nach einer der vorstehenden Verfahrensausführungsformen, wobei das Verfahren weiterhin umfasst:
Erhöhen der CO₂-Konzentration im Nutzraum des CO₂-Inkubators, wenn der CO₂-Wert unterschritten wird.

V12. Verfahren nach einer der vorstehenden Verfahrens-Ausführungsformen, wobei das Verfahren das Empfangen des CO₂-Wertes umfasst.

V13. Verfahren nach einer der vorstehenden Verfahrens-Ausführungsformen, wobei das Verfahren das Steuern oder Regeln der CO₂-Konzentration in Abhängigkeit des CO₂-Wertes umfasst.

V14. Verfahren nach der vorstehenden Verfahrens-Ausführungsform und mit den Merkmalen der Verfahrens-Ausführungsform V11, wobei das Regeln der CO₂-Konzentration das Bestimmen, Vergleichen, Erhöhen und Reduzieren der CO₂-Konzentration im Nutzraum umfasst.

V15. Verfahren nach einer der vorstehenden Verfahrens-Ausführungsformen, wobei der CO₂-Inkubator ein CO₂-Inkubator nach einer der vorstehenden Inkubator-Ausführungsformen ist.

I40. Inkubator nach einer der vorstehenden Inkubator-Ausführungsformen, wobei der Inkubator ausgelegt ist das Verfahren nach einer der vorstehenden Verfahrens-Ausführungsformen durchzuführen.

I41. Inkubator nach einer der vorstehenden Inkubator-Ausführungsformen und mit den Merkmalen von I24, wobei der Controller ausgelegt ist das Verfahren nach einer der vorstehenden Verfahrens-Ausführungsformen durchzuführen.

Ausführungsformen der vorliegenden Erfindung werden nun unter Bezugnahme auf die beigefügten Zeichnungen beschrieben. Diese Ausführungsformen sollen die vorliegende Erfindung nur beispielhaft darstellen, aber nicht einschränken.

Ausführungsformen der vorliegenden Erfindung werden nun unter Bezugnahme auf die beigefügten Zeichnungen beschrieben. Diese Ausführungsformen sollen die vorliegende Erfindung nur beispielhaft darstellen, aber nicht einschränken.
- Fig. 1: zeigt eine erfindungsgemäße Ausführungsform eines CO₂-Inkubators;
- Fig. 2: zeigt eine erfindungsgemäße Ausführungsform eines CO₂-Inkubators bei der aktiv Luft aus dem Nutzraum abgeführt werden kann;
- Fig. 3: zeigt eine erfindungsgemäße Ausführungsform eines CO₂-Inkubators bei der aktiv Luft in den Nutzraum gefördert werden kann;
- Fig. 4: zeigt eine weitere erfindungsgemäße Ausführungsform eines CO₂-Inkubators bei der aktiv Luft in den Nutzraum gefördert werden kann; und
- Fig. 5: illustriert ein erfindungsgemäßes Verfahren zur Änderung der CO₂-Konzentration im Nutzraum eines CO₂-Inkubators.

Es wird darauf hingewiesen, dass nicht alle Zeichnungen alle Bezugszeichen tragen. Stattdessen sind in einigen der Zeichnungen einige der Bezugszeichen der Kürze und Einfachheit der Darstellung halber weggelassen worden. Ausführungsformen der vorliegenden Erfindung werden nun unter Bezugnahme auf die beigefügten Zeichnungen beschrieben.

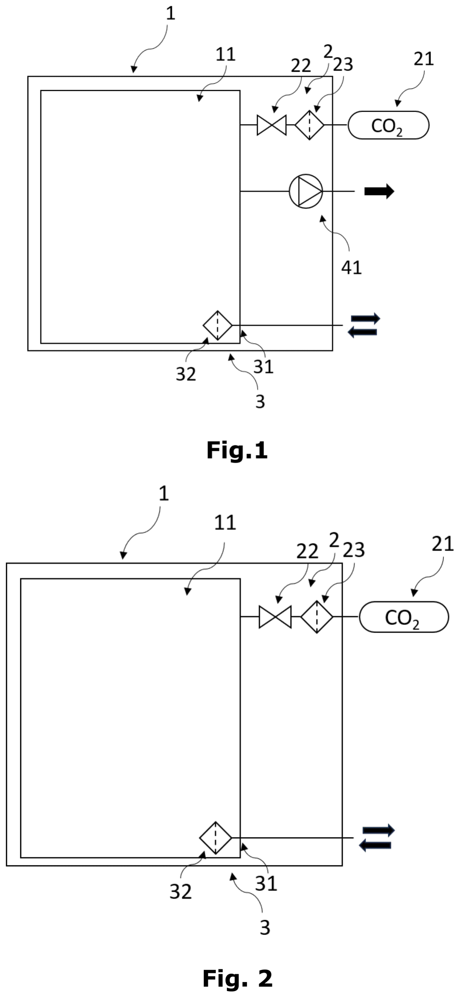

Mit Bezug auf Fig. 1 betrifft die vorliegende Erfindung einen CO₂-Inkubator 1 (im Folgenden auch einfach Inkubator 1), der einen Nutzraum 11, eine CO₂-Versorgung 2 und eine Druckausgleichsöffnung 3 umfasst, wobei die Druckausgleichsöffnung 3 ausgelegt ist, den Druck im Nutzraum 11 an einen Umgebungsdruck des Inkubators 1 anzupassen. Allgemein ist der CO₂-Inkubator 1 ausgelegt, eine CO₂-Konzentration im Nutzraum 11 zu reduzieren.

Der Nutzraum 11 ist typischerweise über mindestens eine Tür des Inkubators 1 zugänglich und kann als einfache Kammer ausgestaltet sein. In dem Nutzraum 11 können dann beispielsweise Behälter mit Zellen untergebracht werden um diese kontrollierten Umweltbedingungen oder Umgebungsbedingungen auszusetzen, die z.B. das Zellwachstum fördern können.

Die CO₂-Versorgung 2 kann dazu ausgelegt sein mit einem CO₂-Reservoir 21 verbunden zu werden, z.B. eine mit CO₂ gefüllte Gasflasche. Das CO₂-Reservoir 21 kann dazu ausgelegt sein, CO₂ bei einem bestimmten Druck zur Verfügung zu stellen, z.B. mit maximal 1 bar über atmosphärischen Druck. Das CO₂-Reservoir 21 kann beispielsweise auch durch eine entsprechende Zuleitung bereitgestellt werden. In manchen Ausführungsformen kann das CO₂-Reservoir auch vom Inkubator 1 umfasst werden.

Die CO₂-Versorgung 2 kann ein CO₂-Ventil 22 umfassen, welches eine Verbindung zwischen dem CO₂-Reservoir 21 und dem Nutzraum 11 wahlweise öffnen oder schließen kann.

Insbesondere kann das CO₂-Ventil 22 vorzugweise ein steuer- bzw. regelbares Ventil sein. Vorzugsweise kann das CO₂-Ventil 22 zusätzlich zu einer geöffneten und einer geschlossenen Position auch teilweise geöffnete Positionen einnehmen um dadurch die CO₂-Menge und/oder den Druck zu steuern oder zu regeln. In anderen Worten kann das CO₂-Ventil 22 ein Proportionalventil sein. Außerdem kann ein CO₂-Filter 23 vorgesehen sein. Dieser Filter kann vorzugsweise dazu ausgelegt sein, dass eingeleitete CO₂ zu filtern. Insbesondere können Verunreinigungen in Form von Partikeln und auch andere Materialien, z.B. Öle aus den Versorgungsleitungen, aus dem Gasstrom entfernt und abgeschieden werden. Der CO₂-Filter 23 kann beispielsweise als Membranfilter bzw. als Sterilfilter aus PTFE ausgeführt werden.

Die Druckausgleichsöffnung 3 umfasst eine fluidische Verbindung 31 zwischen dem Nutzraum und der Umgebung des Inkubators oder alternativ einem auf Umgebungsdruck gehaltenem Reservoir. Diese fluidische Verbindung 31 kann beispielsweise durch eine entsprechende Öffnung oder eine Leitung bereitgestellt werden. Zusätzlich umfasst die Druckausgleichsöffnung 3 vorzugsweise einen Druckausgleichsfilter 32. Der Druckausgleichsfilter 32 kann vorzugsweise ein Sinterfilter sein. Der Sinterfilter kann eine relativ große Porengröße aufweisen, z.B. ca. 200 µm. Der Druckausgleichfilter 32 wirkt im Wesentlichen wie ein Strömungswiderstand. Wird also durch die CO₂-Versorgung 2 CO₂ in den Nutzraum 11 eingelassen, so steigt der Druck im Nutzraum zeitweise an - beispielsweise auf 1,3 atm, während der Umgebungsdruck 1,0 atm beträgt. Über die Druckausgleichöffnung 3 wird in diesem Fall Gas aus dem Nutzraum 11 an die Umgebung abgegeben, wobei der momentane Gasfluss zwischen dem Nutzraum 11 und der Umgebung von der Druckdifferenz zwischen dem Nutzraum 11 und der Umgebung abhängt. Der Druckausgleichsfilter 32 verhindert vorteilhaft, dass ein ungehemmter und unkontrollierter Luftaustausch zwischen Nutzraum und der Umgebung stattfindet - da er als Strömungswiderstand wirkt, ist der Gasaustausch geringer als es der Fall sein würde, wenn kein Druckausgleichfilter 32 vorgesehen wäre. Wird der Druck im Nutzraum erhöht oder gesenkt, kann entsprechend ein stärker Austausch mit der Umgebungsluft bewirkt werden. Der Druckausgleichsfilter 32 kann dazu ausgelegt sein, Staub und grobe Verunreinigungen aus durchströmender Luft zu filtern.

Grundsätzlich ist ein Inkubator zwar nicht völlig dicht, sodass auch ohne eine entsprechende Druckausgleichsöffnung 3 ein gewisser Luftaustausch stattfinden könnte, dieser wäre jedoch zum einen ungefiltert und zum anderen besteht nachteilig das große Risiko, dass bspw. ungewünschte Kondensation an der Tür des Inkubators auftreten kann.

Wird dem Nutzraum 11 mittels der CO₂-Versorgung CO₂ zugeführt, d.h. insbesondere das CO₂-Ventil 22 geöffnet, ist der Druck im Nutzraum 11 zunächst gegenüber dem Umgebungsdruck erhöht. Insgesamt kann so die CO₂-Konzentration im Nutzraum 11 erhöht werden, da CO₂ eingeleitet wird und über die Druckausgleichsöffnung 3 ein Luft- bzw. Gasgemisch aller im Nutzraum vorhandener Gase (und ggf. Aerosole) aus dem Nutzraum entweicht.

Wird das CO₂-Ventil 22 geschlossen, gleicht sich der Druck innerhalb des Nutzraums 11 über die Druckausgleichsöffnung 3 an den Umgebungsdruck an. Ein weiterer Luftaustausch zwischen Nutzraum 11 und Umgebung über die Druckausgleichsöffnung 3 ist durch den Strömungswiderstand des Druckausgleichsfilters gehemmt.

Es versteht sich, dass der erfindungsgemäße CO₂-Inkubator 1 zusätzlich Merkmale aufweisen kann. Insbesondere kann der Inkubator zusätzlich dazu ausgelegt sein, die Temperatur im Nutzraum zu steuern oder zu regeln. Hierzu kann der Inkubator beispielsweise ein Temperierelement und einen Temperatursensor umfassen. Ebenso kann der Inkubator dazu ausgelegt sein, die Feuchte im Nutzraum zu steuern oder zu regeln, z.B. über ein Befeuchtungssystem 5.

Es versteht sich, dass der Inkubator außerdem dazu ausgelegt sein kann die CO₂-Konzentration im Nutzraum zu erhöhen. Insbesondere kann der Inkubator dazu ausgelegt sein, die CO₂-Konzentration im Nutzraum zu steuern oder zu regeln.

Allgemein ist der erfindungsgemäße CO₂-Inkubator dazu ausgelegt, die CO₂-Konzentration im Nutzraum 11 zu reduzieren. Dies kann grundsätzlich realisiert werden, indem Zuluft aktiv in den Nutzraum 11 zugeführt wird, entweder durch das direkte Einbringen von Zuluft oder indem aktiv Luft (oder allgemeiner Gas) aus dem Nutzraum 11 abgesaugt wird, die dann durch nachströmende Zuluft ersetzt wird. Nachfolgend werden entsprechende beispielhafte Ausführungsformen beschrieben.

Figur 2 zeigt eine Ausführungsform, bei der aktiv Luft aus dem Nutzraum 11 abgeführt werden kann. Hierzu kann eine Pumpe 41 vorgesehen sein, die Luft aus dem Nutzraum 11 in die Umgebung fördern kann.

In der gezeigten Ausführungsform kann einerseits die CO₂-Konzentration in dem Nutzraum 11 mittels der CO₂-Versorgung 2 erhöht werden (wie vorstehend erläutert) und andererseits kann die CO₂-Konzentration im Nutzraum mittels Pumpe 41 reduziert werden. Hierzu kann die Pumpe 41 Luft bzw. Gas aus dem Nutzraum 11 in die Umgebung des CO₂-Inkubators 1 fördern. Vorzugsweise ist vorgesehen, dass die Pumpe 41 nur bei geschlossenem CO₂-Ventil 22 läuft. Der im Nutzraum 11 entstehende Unterdruck im Vergleich zur Umgebung wird dann über die Druckausgleichsöffnung 3 ausgeglichen, über die Zuluft (vorzugsweise Umgebungsluft) in den Nutzraum einströmt. Dies ermöglicht vorteilhaft die CO₂-Konzentration im Nutzraum zu reduzieren, solange die CO₂-Konzentration in der Zuluft unterhalb der CO₂-Konzentration im Nutzraum und bei entsprechender Steuerung unterhalb des gewünschten Sollwertes liegt. Beispielsweise liegt der CO₂-Gehalt in der Atmosphäre bei ca. 0,04 % oder 400 ppm, kann in geschlossenen Räumen je nach Belüftung jedoch auch höher liegen (z.B. bis zu 1000 ppm oder auch mehr).

In anderen Worten kann die CO₂-Konzentration durch Absaugen von Luft bzw. Gas aus dem Inkubator und genauer aus dem Nutzraum 11 des Inkubators verringert werden. Dies kann durch eine (Vakuum-/Luft-)Pumpe 41 erfolgen. Alternativ kann der Nutzraum auch über ein Ventil mit einem Bereich mit Unterdruck verbunden werden, um so Luft aus dem Nutzraum abzusaugen. Die Pumpe 41 oder das Ventil können durch einen elektronischen Soll-Ist-Vergleich gesteuert oder bei Überschreitung eines CO₂-Grenzwertes ausgelöst werden. Die Überschreitung eines CO₂-Grenzwertes kann beispielsweise einen CO₂-Alarm auslösen.

Alternativ kann der Nutzraum 11 auch über ein Abluftventil fluidisch mit einem Abluftreservoir verbunden sein, das einen geringeren Druck als den Umgebungsdruck aufweist. Wird das Abluftventil geöffnet, strömt folglich Luft aus dem Nutzraum in das Abluftreservoir.

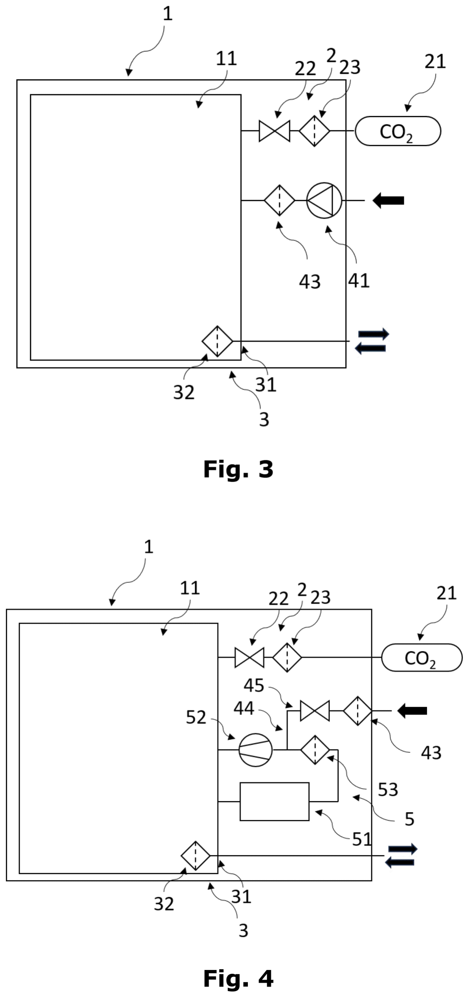

Figur 3 zeigt eine weitere Ausführungsform, bei der aktiv Zuluft in den Nutzraum 11 gefördert werden kann. Hierzu kann eine Pumpe 41 vorgesehen sein, die Luft aus einem Zuluftreservoir in den Nutzraum fördert. Das Zuluftreservoir kann bevorzugt durch die Umgebung des CO₂-Inkubators 1 oder aber auch durch ein Gasreservoir, z.B. eine Gasflasche, bereitgestellt werden. Dies kann ermöglichen Umgebungsluft oder alternativ ein Gas oder Gasgemisch mit kontrollierter Zusammensetzung, z.B. aus Stickstoff und Sauerstoff, in den Nutzraum zu fördern. Ähnlich zu der Ausführungsform in Fig. 2 ist vorzugsweise vorgesehen, dass die Pumpe 41 nur bei geschlossenem CO₂-Ventil 22 läuft. Der im Nutzraum 11 entstehende Überdruck im Vergleich zur Umgebung wird dann über die Druckausgleichsöffnung 3 ausgeglichen, über die Luft bzw. Gas aus dem Nutzraum in die Umgebung austritt. Dies ermöglicht vorteilhaft, die CO₂-Konzentration im Nutzraum zu reduzieren, solange die CO₂-Konzentration in der Zuluft unterhalb der CO₂-Konzentration im Nutzraum und bei entsprechender Steuerung unterhalb des gewünschten Sollwertes liegt.

Um Verunreinigungen durch die zugeführte Zuluft zu vermeiden, kann weiterhin ein Zuluftfilter 43 vorgesehen sein, der die zugeführte Zuluft vorzugsweise stromabwärts der Pumpe 41 filtert. Der Zuluftfilter 43 kann beispielsweise einen Steril- und/oder Membranfilter umfassen. Dadurch können Verunreinigungen des Nutzraums durch die hinzugeführte Zuluft vorteilhaft vermieden werden.

In anderen Worten wird Zuluft in den Inkubator und genauer den Nutzraum 11 eingeblasen. Dazu wird eine (Luft-)Pumpe 41 verwendet, die Luft von außerhalb des CO₂-Inkubators durch einen Zuluftfilter 43, der die Zuluft reinigt, in den Nutzraum 11 pumpt. Die Pumpe kann bei der Überschreitung eines CO₂-Grenzwertes (z.B. einem CO₂-Alarm) ausgelöst oder durch einen elektronischen Soll-Ist-Vergleich gesteuert werden.

Figur 4 zeigt eine weitere Ausführungsform, bei der aktiv Zuluft in den Nutzraum 11 gefördert werden kann. In dieser Ausführungsform wird die Zuluft bei Bedarf über einen vom CO₂-Inkbuator umfassten Luftkanal 5 eingebracht.

Der Luftkanal 5 kann allgemein zur Zirkulation und optional Befeuchtung der Luft im Nutzraum vorgesehen sein. Hierzu kann der Luftkanal einen Einlass und einen Auslass umfassen, die jeweils mit dem Nutzraum verbunden sind und einen Ventilator 52 (auch Lüfter 52 genannt), der dazu ausgelegt ist, Luft vom Einlass zum Auslass des Luftkanals 5 zu fördern.

Weiterhin kann der Luftkanal 5 einen Strömungswiderstand 53 umfassen, der stromaufwärts des Ventilators 52 und stromabwärts des Einlasses angeordnet ist. Der Strömungswiderstand 53 kann vorzugsweise einen Filter 53 umfassen, vorzugsweise einen Schwebstofffilter 53, z.B. einen HEPA-Filter. Durch Luftwiderstand, der vom Strömungswiderstand 53 bereitgestellt wird, kommt es zu einem Druckabfall über den Strömungswiderstand 53 (der Druck vor, d.h. stromaufwärts des Strömungswiderstandes 53 ist also höher als der Druck hinter, d.h. stromabwärts des Strömungswiderstandes). Bei entsprechender Auslegung des Ventilators 52 kann also ein Unterdruck zwischen Ventilator 52 und Strömungswiderstand 53 entstehen - der Druck zwischen diesen Einheiten (also stromabwärts des Widerstandes 53 und stromaufwärts des Ventilators 52) ist also kleiner als der Druck stromabwärts des Ventilators. Dieser Unterdruck kann vorteilhaft genutzt werden, um Zuluft in den Nutzraum 11 einzubringen. Der Unterdruck kann beispielsweise bis zu 1 mbar unter atmosphärischem Druck betragen.

Hierzu kann zwischen Strömungswiderstand 53 und Ventilator 52 eine Zuluftleitung 44 vorgesehen sein, über die Zuluft angesaugt werden kann. Die Zuluftleitung 44 kann vorteilhaft ein Zuluftventil 45 umfassen, welches die Zuluftleitung 44 öffnen und schließen kann. Vorteilhaft ist das Zuluftventil 45 ein Proportionalventil, welches auch Zwischenstellungen einnehmen kann. Weiterhin kann auch die Zuluftleitung 44 einen Zuluftfilter 43 umfassen, der die zugeführte Zuluft filtert um eine Kontamination des Nutzraums 11 zu vermeiden.

Der Luftkanal 5 kann einen Befeuchter 51 bzw. Vernebler 51 umfassen, der Wasser aus einem Wasserreservoir verdampft und/oder vernebelt um die relative Feuchte im Nutzraum zu erhöhen. Vorzugsweise können der Ventilator 52 und der Strömungswiderstand 53 stromabwärts von dem Befeuchter 51 angeordnet sein und ausgelegt sein Luft aus dem Nutzraum 11 über den Befeuchter 51 anzusaugen und wieder in den Nutzraum einzublasen.

In anderen Worten kann Zuluft durch das Inkubator eigene Luftkanalsystem in den Nutzraum 11 gesaugt werden. Dies ist kann dadurch ermöglicht werden, dass ein Strömungswiderstand (vorzugsweise ein Schwebstofffilter) 53, insbesondere ein HEPA-Filter, vor einem Ventilator 52 angeordnet wird, der Luft aus dem Nutzraum 11 durch den Strömungswiderstand 53 ansaugt, wodurch ein Unterdruck zwischen dem Strömungswiderstand 53 und dem Ventilator 52 entsteht. Der Unterdruck wird durch den Druckabfall über dem HEPA- Strömungswiderstand 53 verursacht, der proportional zum Volumenstrom durch den Strömungswiderstand ist und zwischen 0 und -1mbar liegen kann (in Bezug auf den atmosphärischen Druck). Um zu verhindern, dass Verunreinigungen oder Partikel in den Nutzraum gelangen, kann Zuluft über einen externen Zuluftfilter 43 (z.B. einen Membranfilter) angesaugt und bspw. in ein Filtergehäuse des Schwebstofffilters 53 geleitet werden. Die korrekte Menge der angesaugten Luft kann über einen Druckabfall des externen Zuluftfilters 43 und ggf. über eine zusätzliche Kapillare in der Zuleitung 44 eingestellt werden. Durch den Einbau eines Zuluftventils 45 zwischen Zuluftfilter 43 und dem Unterdruckbereich zwischen Strömungswiderstand 53 und Ventilator 52, z.B. dem Filtergehäuse des Filters 53, bspw. bereitgestellt durch einem Schlauch, der an der HEPA-Filter-Airbox angeschlossen ist, kann die Luftansaugung gesteuert und/oder geregelt werden. Zum Beispiel kann die Luftansaugung durch Überschreitung eines CO₂-Grenzwertes (wobei ein CO₂-Alarm ausgelöst werden kann) ausgelöst oder durch einen elektronischen Soll-Ist-Vergleich gesteuert werden.

Diese Ausführungsform nutzt vorteilhaft einen ohnehin typischerweise bereits bestehende Luftkanal 5 (bspw. als Teil eines Befeuchtungssystem), wodurch im Vergleich zu den Ausführungsformen in Figs. 2 und 3 auf eine zusätzliche Pumpe 41 verzichtet werden kann. Im Vergleich zur Verwendung einer zusätzlichen Pumpe kann jedoch nur eine geringes Luftvolumen pro Zeit eingebracht werden, wohingegen eine zusätzliche Pumpe 41 größere Volumen pro Zeit fördern kann.

Die Zuluft kann vorteilhaft Umgebungsluft des Inkubators 1 sein, d.h. etwaige Zuluftreservoirs können durch die Umgebung des CO₂-Inkubators bereitgestellt werden. Alternativ können auch Zuluftreservoirs verwendet werden, die ein vorbestimmtes Gas oder Gasgemisch umfassen.

Grundsätzlich kann der CO₂-Inkubator einen Controller umfassen, der die CO₂-Konzentration im Nutzraum bspw. mittels entsprechendem CO₂-Sensor überwachen und ggf. regeln oder bevorzugt steuern kann. Hierzu kann der Controller Daten mit mindestens einem CO₂-Sensor, dem CO₂-Ventil 22, und je nach Ausführungsform der Pumpe 41, dem Zuluftventil 45 und/oder dem Ventilator 52 austauschen um Daten auszulesen und/oder diese zu regeln oder zu steuern. Dadurch kann der Controller bspw. bei Überschreitung eines CO₂-Grenzwertes Maßnahmen zur Reduzierung der CO₂-Konzentration einleiten und ggf. einen CO₂-Alarm auslösen oder alternativ die CO₂-Konzentration aktiv mittels Ist-Sollwert-Vergleich steuern.

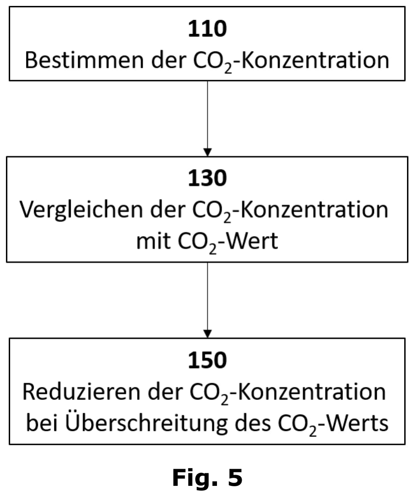

Mit Bezug auf Fig. 5 betrifft die vorliegende Erfindung auch ein entsprechendes Verfahren zur Änderung der CO₂-Konzentration im Nutzraum eines CO₂-Inkubators. Dieses Verfahren umfasst das Bestimmen der CO₂-Konzentration im Nutzraum, das Vergleichen der CO₂-Konzentration mit einem vorgegebenen CO₂-Wert und das Reduzieren der CO₂-Konzentration im Nutzraum des CO₂-Inkubators, wenn der CO₂-Wert überschritten wird.

Der CO₂-Wert kann dabei ein CO₂-Grenzwert sein oder auch ein CO₂-Sollwert. Der CO₂-Grenzwert kann ermöglichen eine zu hohe CO₂-Konzentration festzustellen, ggf. einen CO₂-Alarm auszulösen und direkt automatisch entsprechende Maßnahmen zur Reduzierung einzuleiten. Der CO₂-Sollwert ermöglicht eine Steuerung der CO₂-Konzentration durch Abgleich mit einem durch einen CO₂-Sensor bestimmten Istwert der CO₂-Konzentration im Nutzraum.

Das Verfahren kann das Empfangen des CO₂-Wertes umfassen. Beispielsweise kann der CO₂-Wert durch einen Nutzer vorgegeben werden.

Der Schritt des Reduzierens der CO₂-Konzentration kann das aktive Zuführen von Zuluft in den Nutzraum umfassen, entweder durch das Absaugen von Luft bzw. Gas aus dem Nutzraum, sodass Zuluft bspw. über eine Druckausgleichsöffnung nachströmt, oder durch Pumpen von Zuluft in den Nutzraum. Alternativ kann auch Zuluft ähnlich wie CO₂ aus einem Zuluftreservoir über ein Ventil eingeleitet werden, wobei das Zuluftreservoir Zuluft bei einem relativ zum Nutzraum erhöhten Druck bereitstellt.

Das Verfahren kann außerdem das Erhöhen der CO₂-Konzentration im Nutzraum umfassen, wenn der CO₂-Wert unterschritten wird. Die Erhöhung der CO₂-Konzentration kann wie oben beschrieben über das Zuführen von CO₂ aus einem CO₂-Reservoir erfolgen, welches CO₂ bei einem relativ zum Nutzraum erhöhten Druck bereitstellen kann, zum Beispiel durch das Öffnen eines CO₂-Ventils, welches zwischen Nutzraum und CO₂-Reservoir angeordnet ist.

Insbesondere kann das Verfahren das Regeln und vorzugsweise das Steuern der CO₂-Konzentration in Abhängigkeit des CO₂-Wertes umfassen, wobei das Steuern der CO₂-Konzentration das Bestimmen, Vergleichen, Erhöhen und Reduzieren der CO₂-Konzentration im Nutzraum umfasst.

Die vorliegende Erfindung ermöglicht somit vorteilhaft automatisch eine zu hohe CO₂-Konzentration im Inkubator zu reduzieren und kann so Fehlernachrichten vermeiden, die bei bekannten Geräten typischerweise ausgegeben werden. Insbesondere muss der Nutzer nicht selbst eingreifen und beispielsweise manuell die Tür öffnen.

Wenn in dieser Beschreibung oder den Ansprüchen ein relativer Begriff wie "ungefähr", "im Wesentlichen" oder "annähernd" verwendet wird, sollte ein solcher Begriff auch so ausgelegt werden, dass er den exakten Begriff einschließt. Das heißt, z. B. "im Wesentlichen gerade" sollte so ausgelegt werden, dass er auch "(genau) gerade" einschließt.

Wann immer in den obigen oder auch in den beigefügten Ansprüchen Schritte rezitiert wurden, ist zu beachten, dass die Reihenfolge, in der die Schritte in diesem Text rezitiert werden, zufällig sein kann. Das heißt, dass die Reihenfolge, in der die Schritte rezitiert werden, zufällig sein kann, es sei denn, es wird etwas anderes angegeben oder es ist für den Fachmann klar. Das heißt, wenn im vorliegenden Dokument z. B. angegeben wird, dass ein Verfahren die Schritte (A) und (B) umfasst, bedeutet dies nicht notwendigerweise, dass Schritt (A) vor Schritt (B) erfolgt, sondern es ist auch möglich, dass Schritt (A) (zumindest teilweise) gleichzeitig mit Schritt (B) ausgeführt wird oder dass Schritt (B) vor Schritt (A) erfolgt. Wenn außerdem gesagt wird, dass ein Schritt (X) einem anderen Schritt (Z) vorausgeht, bedeutet dies nicht, dass es keinen Schritt zwischen den Schritten (X) und (Z) gibt. Das heißt, Schritt (X) vor Schritt (Z) umfasst die Situation, dass Schritt (X) direkt vor Schritt (Z) ausgeführt wird, aber auch die Situation, dass (X) vor einem oder mehreren Schritten (Y1), ..., gefolgt von Schritt (Z), ausgeführt wird. Entsprechende Überlegungen gelten, wenn Begriffe wie "nach" oder "vor" verwendet werden.

Während im Vorstehenden eine bevorzugte Ausführungsform unter Bezugnahme auf die begleitenden Zeichnungen beschrieben wurde, wird der Fachmann verstehen, dass diese Ausführungsform nur zu Veranschaulichungszwecken bereitgestellt wurde und keinesfalls als Einschränkung des Umfangs der vorliegenden Erfindung, die durch die Ansprüche definiert ist, ausgelegt werden sollte.

## Patentansprüche

1. CO₂-Inkubator umfassend
einen Nutzraum (11),
eine Druckausgleichsöffnung (3), die dazu ausgelegt ist, den Druck im Nutzraum (11) an den Umgebungsdruck anzupassen, und
eine CO₂-Versorgung (2),
wobei der CO₂-Inkubator dazu ausgelegt ist, eine CO₂-Konzentration im Nutzraum (11) zu reduzieren.

2. Inkubator nach dem vorstehenden Anspruch, wobei die Druckausgleichsöffnung (3) einen Druckausgleichsfilter (32) umfasst, der vorzugsweise einen Sinterfilter umfasst.

3. Inkubator nach einem der vorstehenden Ansprüche, wobei der Inkubator dazu ausgelegt ist aktiv Luft aus dem Nutzraum (11) abzusaugen und dadurch über die Druckausgleichsöffnung (3) Zuluft zuzuführen.

4. Inkubator nach einem der vorstehenden Ansprüche, wobei der wobei der Inkubator eine Pumpe (41) umfasst und wobei die Pumpe (41) dazu ausgelegt ist Luft aus dem Nutzraum (11) abzusaugen.

5. Inkubator nach einem der Ansprüche 1 oder 2, wobei der Inkubator dazu ausgelegt ist Zuluft aktiv in den Nutzraum (11) einzubringen.

6. Inkubator nach einem der Ansprüche 1, 2 oder 5, wobei der wobei der Inkubator eine Pumpe (41) umfasst und wobei die Pumpe (41) dazu ausgelegt ist Zuluft in den Nutzraum (11) zu fördern.

7. Inkubator nach einem der vorstehenden Ansprüche, wobei der Inkubator einen Luftkanal (5) umfasst, wobei der Luftkanal (5) umfasst:
einen Einlass und einen Auslass, die jeweils mit dem Nutzraum (11) verbunden sind,
einen Ventilator (52), der dazu ausgelegt ist Luft vom Einlass zum Auslass des Luftkanals (5) zu fördern, und
einem Strömungswiderstand (53), der stromaufwärts des Ventilators (52) und stromabwärts des Einlasses angeordnet ist;
wobei zwischen dem Strömungswiderstand (53) dem Ventilator (52) eine Zuluftleitung (44) angeordnet ist, die mit einem Zuluftreservoir verbunden ist.

8. Inkubator nach einem der vorstehenden Ansprüche, wobei der Inkubator einen Controller umfasst, und wobei der Controller dazu ausgelegt ist,
die CO₂-Konzentration im Nutzraum (11) zu reduzieren, wenn ein CO₂-Grenzwert überschritten wird, und/oder
die CO₂-Konzentration im Nutzraum (11) bezüglich eines vorgegebenen Sollwertes zu steuern und/oder zu regeln.

9. Verfahren zur Änderung einer CO₂-Konzentration in einem Nutzraum eines CO₂-Inkubators, wobei das Verfahren umfasst:
Bestimmen der CO₂-Konzentration im Nutzraum,
Vergleichen der CO₂-Konzentration mit einem vorgegebenen CO₂-Wert,
Reduzieren der CO₂-Konzentration im Nutzraum des CO₂-Inkubators, wenn der CO₂-Wert überschritten wird.

10. Verfahren nach dem vorstehenden Anspruch, wobei das Reduzieren der CO₂-Konzentration im Nutzraum das aktive Zuführen von Zuluft in den Nutzraum umfasst.

11. Verfahren nach dem vorstehenden Anspruch, wobei das aktive Zuführen von Zuluft das Ansaugen von Zuluft über eine mit einem Luftkanal des Inkubators verbundenen Zuluftleitung mittels Unterdruck umfasst.

12. Verfahren nach Anspruch 10, wobei das aktive Zuführen von Zuluft das Absaugen von Luft aus den Nutzraum umfasst, um so Zuluft beispielsweise über eine Druckausgleichsöffnung nachströmen zu lassen.

13. Verfahren nach einem der Ansprüche 9bis 12, wobei das Verfahren weiterhin umfasst:
Erhöhen der CO₂-Konzentration im Nutzraum des CO₂-Inkubators, wenn der CO₂-Wert unterschritten wird.

14. Verfahren nach dem vorstehenden Anspruch, wobei das Verfahren das Regeln der CO₂-Konzentration in Abhängigkeit des CO₂-Wertes umfasst und wobei das Steuern der CO₂-Konzentration das Bestimmen, Vergleichen, Erhöhen und Reduzieren der CO₂-Konzentration im Nutzraum umfasst.

15. Verfahren nach einem der Ansprüche 9 bis 14, wobei der CO₂-Inkubator ein CO₂-Inkubator nach einem der Ansprüche 1 bis 8 ist.
